## Europäisches Patentamt

## European Patent Office

## Office européen des brevets

(11) Publication number: **0 003 374**
**B1**

(12) # EUROPEAN PATENT SPECIFICATION

(45) Date of publication of patent specification: **12.08.81**

(51) Int. Cl.³: **C 07 D 251/50**

(21) Application number: **79200023.4**

(22) Date of filing: **12.01.79**

(54) Process for the preparation of a substituted triazine.

(30) Priority: **23.01.78 US 871656**

(43) Date of publication of application:
**08.08.79 Bulletin 79/16**

(45) Publication of the grant of the European patent:
**12.08.81 Bulletin 81/32**

(84) Designated Contracting States:
**CH DE FR GB IT NL**

(56) References cited:
**DE - A - 2 032 861**
**US - A - 3 681 337**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Colby, Thomas Harold**
**2622 Pomeran Drive**
**Houston Texas 77055 (US)**
Inventor: **Freitas, Ernest Robert**
**2115 Fulham Court**
**Houston Texas 77042 (US)**
Inventor: **Durland, William Ross**
**13714 Alchester**
**Houston Texas 77024 (US)**

(74) Representative: **Keuzenkamp, Abraham et al,**
**P.O. Box 302**
**NL-2501 CH Den Haag (NL)**

Courier Press, Leamington Spa, England.

# 0 003 374

Process for the preparation of a substituted triazine.

This invention relates to a process for the preparation of 2 - chloro - 4 - ethylamino - 6 - isopropylamino - s - triazine.

2 - chloro - 4 - ethylamino - 6 - isopropylamino - s - triazine, proprietary name "atrazine", is a selective herbicide for weed control among cultivated crops, especially maize. Although other synthetic routes are possible, much, if not all, of the commercial atrazine production is carried out by the stepwise replacement of chlorine atoms in cyanuric chloride by reaction with the appropriate monoalkyl amines in the presence of an acid binding agent, typically an alkali metal hydroxide. Various solvent systems have been proposed for use in this process: a particularly advantageous solvent because of availability and ease of handling, is mixed acetone and water.

In this conventional process, which may be carried out using batchwise or continuous techniques, cyanuric chloride is initially reacted with isopropylamine to form a 2,4 - dichloro - 6 - isopropylamino - s - triazine intermediate and this intermediate is subsequently reacted, without purification or other treatment, with ethylamine to form the desired product. Both amination reactions are carried out in the presence of an alkali metal hydroxide acid binding agent in a mixed acetone/water solvent system. The mixed acetone/water solvent system employed in this conventional process typically contains from about 60 to 90% by weight acetone; the exact solvent composition may vary between reaction stages depending on the amount of water added in with the acid binding agent, which is suitably added to the reaction mixture throughout the reaction as an aqueous solution.

Although the state of the art on the synthesis of substituted alkylamino - s - triazine herbicides from cyanuric chloride is rather advanced, commercial atrazine production units are not devoid of problems. One problem is the occurrence of side reactions during the atrazine synthesis which lead to alkylamino substituted s - triazine by-products, for example tris(alkylamino) - s - triazines, 2 - chloro - 4,6 - bis(ethylamino) - s - triazine and 2 - chloro - 4,6 - bis(isopropylamino) - s - triazine in the desired triazine product. These by-products must generally be held to rather low levels in the final product to ensure consistent biological performance and handling properties in the field. Typically, the specification for technical atrazine requires that neither one of the latter two impurities should exceed one percent by weight of the technical product. Generally, this specification can be met by carefully controlling the molar ratio of triazine compound to ethylamine and to isopropylamine, at or very close to the stoichiometric ratio, in eac h of the two amination reaction steps. In this connection reference is made to German Patent Application 2,032,861. While this measure of control is possible in the laboratory, it is very difficult to maintain adequate control on a commercial scale, especially when using an acetone/water solvent system. The use of a small molar excess of isopropylamine in the first stage of the synthesis leads to the formation of 2 - chloro - 4,6 - bis(isopropylamino) - s - triazine, whereas the use of less than the stoichiometric amount of isopropylamine gives rise to the formation of 2 - chloro - 4,6 - bis(ethylamino) - s - triazine in the second reaction stage. Small deviations from the stoichiometric ratio in the first reaction stage can, depending on the reaction conditions, lead to the formation of amounts of either of these impurities in excess of the amounts permitted in technical atrazine. A process in which especially the formation of tris(alkylamino) - s - triazines is averted is described in US Patent Specification 3,681,337.

The Applicants have now found a simple modification of the conventional process which obviates the need to control the ratio of the reactants to be very close to the stoichiometric ratio.

The present invention provides a process for the preparation of 2 - chloro - 4 - ethylamino - 6 - isopropylamino - s - triazine in which cyanuric chloride is reacted in a first amination step with either isopropylamine or ethylamine to give either 2,6 - dichloro - 4 - isopropylamino - s - triazine or 2,6 - dichloro - 4 - ethylamino - s - triazine respectively, which is subsequently reacted in a second amination step with either ethylamine or isopropylamine respectively, both reactions being performed in a mixed acetone/water solvent and in the presence of an acid binding agent, characterised in that in the first amination step the molar ratio of cyanuric chloride to alkylamine is at least 1.04:1, and that subsequent to the first amination step but prior to the second amination step, the cyanuric chloride remaining in the reaction mixture is hydrolysed, using a sufficient quantity of a base to maintain the pH of the reaction mixture in the range of 6 to 11, to a water-soluble salt of monohydroxydichlorotriazine.

Preferably, the cyanuric chloride is reacted with isopropylamine in the first amination step, and the resulting 2 - chloro - 4 - isopropylamino - s - triazine is reacted with ethylamine in the second amination step, and preferably the acid binding agent is an alkali metal hydroxide.

The use of an excess of cyanuric chloride in the first amination step of the reaction precludes the formation of excessive amounts of 2 - chloro - 4,6 - bis(alkylamino) - s - triazine in the first amination step. The molar excess of cyanuric chloride used is not critical provided that it is at least 4%, but, in order to avoid excessively long reaction times and large losses of cyanuric chloride, the ratio of cyanuric chloride to alkylamine in the first amination step of the reaction is preferably in the range 1.04:1 to 1.08:1.

The excess cyanuric chloride in the reaction product from the first amination step is converted in the subsequent hydrolysis to a basic salt of monohydroxydichlorotriazine, by treating the reaction

2

O 003 374

product with a base under conditions which promote the basic salt formation without significant degradation of the 2,6 - dichloro - 4 - alkylamino - s - triazine intermediate. The hydrolysis may be carried out batchwise or continuously. The reaction mixture from the first amination step is reacted, in the same or a different reaction vessel, with a base, preferably an alkali or alkaline earth metal hydroxide, for a reaction time and at a temperature sufficient to effect substantial conversion of the unreacted cyanuric chloride to the hydroxytriazine salt. The base employed for this hydrolysis reaction is preferably an alkali metal hydroxide and most preferably sodium hydroxide. The base may be added to the reaction mixture in any convenient manner, but is preferably introduced as an aqueous solution, for example an aqueous solution containing 15 to 30% by weight of sodium hydroxide. The amount of base employed should be at least sufficient to react with all the excess cyanuric chloride; preferably a slight excess of base over that required to effect complete conversion of the excess cyanuric chloride present, is used. In the mixed acetone/water solvent system, the desired quantity of base may readily be maintained by adding sufficient base during the hydrolysis reaction to maintain the pH of the reaction mixture at from 6 to 11, preferably from 7 to 10. The hydrolysis reaction is suitably carried out at or near atmospheric pressure, preferably at a reaction temperature of from 35°C to 50°C. Under these conditions, substantially complete hydrolysis of the unreacted cyanuric chloride is generally obtained in reaction times ranging from about 0.5 to about 2 hours.

Base catalysed hydrolysis of the excess cyanuric chloride is more advantageous than acid catalysed hydrolysis, because of the faster reaction rate and the reduced production of other hydrolysis by-products which are prevalent at highly acidic pH's. In fact, with the hydrolysis technique of the present invention, it is possible to hydrolyse a substantial quantity e.g. up to 90%, of the excess cyanuric chloride in reaction times of 2 hours or less without causing significant hydrolysis of the 2,4 - dichloro - 6 - alkylamino - s - triazine intermediate. Further, a large proportion of the basic salt of monohydroxydichlorotriazine which is formed by hydrolysis in the presence of a base remains in the aqueous phase which forms from the mixed solvent system during the first amination step and the subsequent hydrolysis reaction. The partial immiscibility of the system arises primarily as a result of the build-up of the products of the first amination step — i.e. chloride salt and triazine intermediate — in the mixed solvent system. The reaction mixture may be passed directly from the hydrolysis step to the second amination step, but preferably at least some of the basic salt of monohydroxydichlorotriazine is removed from the reaction mixture prior to the second amination step by separating off the aqueous phase. In this way, a significant proportion of the basic salt of monohydroxydichlorotriazine, for example up to about half, can easily be removed from the reaction system thus minimising the more difficult separation problems encountered after the completion of the synthesis reaction scheme when the bulk of the desired 2 - chloro - 4 - ethylamino - 6 - isopropylamino - s - triazine is present as a solid precipitate.

Separation of the two phases may be carried out batch-wise or continuously, and is preferably effected by passing the hydrolysis reaction mixture after the hydrolysis to a separate vessel of adequate size to provide sufficient residence time for adequate separation of the phases. The phase separation step is suitably carried out at a temperature of from 25 to 50°C and a pressure of from 98.07 to 137.29 kPa (1 to 1.4 atmospheres); under these conditions, complete separation of the aqueous phase is generally obtained in 10 to 20 minutes. The aqueous phase which separates as the lower phase in the phase separation vessel will typically contain a minor amount, for example 10 to 20% by weight. of acetone. In addition, from 20 to 50% of the water originally present remains in the acetone phase, which accounts for the incomplete removal of the basic salt of monohydroxydichlorotriazine when the aqueous phase is removed. After removal of the aqueous phase as a bottoms product from the phase separation vessel, the organic phase is adjusted to the temperature desired for the second amination step, and maintained at a pH of from 8 to 9 to avoid hydrolysis of the 2,4 - dichloro - 6 - alkylamino - s - triazine intermediate prior to its reaction in the second amination step. The separated organic phase from the optional phase separation step typically contains 5 to 15% by weight water based on the total weight of solvent, present, and may therefore if desired be used directly in the second amination step without adding additional water, especially where the acid binding agent for the second amination step is added as an aqueous solution.

It will be appreciated that the lower end of the pH range within which the excess cyanuric chloride can be hydrolysed, is actually a slightly acid pH. However, base must be added to the reaction mixture to maintain the pH at even this marginally acidic value in view of the highly acidic nature of free monohydroxydichlorotriazine. Thus, even at pH 6, substantially all of the highly acidic monohydroxydichlorotriazine is present in the form of a basic salt and the benefits of the invention, including high reaction rates, selective hydrolysis and favourable partitioning between aqueous and non-aqueous solvent phases may all be obtained.

The second amination step of the process according to the invention may be carried out using molar ratios of triazine intermediate to alkylamine which approximate the stoichiometric ratio for the reaction. Preferably however a slight excess of alkylamine over triazine intermediate is used, in order to reduce the reaction time and in order to promote substantially complete reaction of the triazine intermediate. For example, the molar ratio of alkylamine to triazine intermediate may be in the range 1:1 to 1.05:1.

3

# O 003 374

The acid binding agent used in each amination step is suitably an alkali metal hydroxide, preferably sodium hydroxide, and is conveniently added to the reaction mixture as an aqueous solution, for example an aqueous solution containing 15 to 30% by weight sodium hydroxide. The acid binding agent acts as a sink for the hydrogen chloride released during the amination reactions, and, in order for the amination reactions to proceed the completion, it must be present in a molar amount at least equal to the number of moles of triazine or alkylamine, whichever is the lower, present. In the mixed acetone/water solvent system, the correct quantity of acid binding agent may readily be maintained by adding sufficient acid binding agent to maintain the pH of the reaction system at about 8 to 9 in both reaction stages.

Both amination reactions may be carried out at atmospheric pressure, but are preferably conducted at reduced pressure, for example at 13.33 to 66.66 kPa (100 to 500 mm Hg). At these reduced pressures, the reaction temperature is suitably in the range from 5 to 60°C, with a temperature in the range of 10 to 15°C being preferred for the first amination step and a temperature in the range of 35°C to 50°C being preferred for the second amination step. Under these conditions the first amination step is usually completed in 1 to 4 hours while the second amination step goes to completion in 0.5 to 6 hours.

The following Examples illustrate the invention. Examples 2 to 4 illustrate processes according to the invention, while Example 1 is for comparative purposes only.

Example 1 — Comparative Example not according to the invention.

A batch synthesis of 2 - chloro - 4 - ethylamino - 6 - isopropylamino - s - triazine was carried out in a mixed acetone/water solvent (45% by weight acetone) using a slight stoichiometric excess of cyanuric chloride in the first amination step — i.e. molar ratio of cyanuric chloride:isopropylamine 1.02:1 — and a molar ratio of 2,4 - dichloro - 4 - isopropylamino - s - triazine:ethylamine 0.95:1 — to ensure complete conversion to the desired triazine intermediate in the first amination step. The stepwise synthesis was conducted in a 2,000 ml reactor equipped with an agitator and efficient baffle by initially charging acetone (400 g), cyanuric chloride (176.4 g, 0.956 moles), and isopropylamine (55.7 g, 0.947 moles) into the reactor and subsequently adding 188.4 g of an aqueous soluton containing 20% by weight of sodium hydroxide over a period of 45 minutes at a reaction temperature and pressure of 10°C and 101 kPa (760 mm Hg), respectively. At the end of the 20% aqueous sodium hydroxide addition, the pH of the reaction mixture was 8.0. After an additional 20 minute reaction time at 10°C under agitation, ethylamine (66.0 g, 0.996 moles) was added in the form of a 68% by weight aqueous solution, and the temperature of the reactor was increased to 35°C. Subsequently 156.4 g of 20% by weight aqueous sodium hydroxide were added to the agitated reactor over a period of 40 minutes while maintaining the reactor at a temperature of 35°C and a pressure of 101.32 kPa (760 mm Hg.). After an additional 35 minute reaction time, the pH was 8.5, and the reactor was cooled and the product was precipitated by the addition of 1.2 litres of water. 188.6 g of crude produce were obtained. Analysis of this product by liquid chromatography gave the following composition:—

| Component | Percent by Weight |
|---|---|
| 2-chloro-4-ethylamino-6-isopropylamino-s-triazine | 92.3 |
| 2-chloro-4,6-bis(ethylamino)-s-triazine | 1.52 |
| 2-chloro-4,6-bis(isopropyl-amino)-s-triazine | 0.59 |

Example 2

The general procedure described in Example 1 was repeated with the following modifications. First, the relative quantities of reactants added to the first reaction were altered to give a cyanuric chloride:isopropylamine molar ratio of 1.04:1 in the first amination step. Secondly, the reaction mixture from the first amination step, containing unreacted cyanuric chloride, was subjected to base hydrolysis prior to addition of the ethylamine for the second amination step. This hydrolysis was carried out in the same reaction vessel used for the amination reactions, by adding sufficient sodium hydroxide, as a 20% by weight aqueous solution, to maintain the pH at 10.5 for a reaction time of 30 minutes at a reaction temperature of 35°C. Upon completion of the hydrolysis reaction period, the second amination step was carried out as in Example 1, and water was added to afford a crude produce whose analysis was as follows:—

| Component | Percent by Weight |
|---|---|
| 2-chloro-4-ethylamino-6-isopropylamino-s-triazine | 98.6 |
| 2-chloro-4,6-bis(ethylamino)-s-triazine | 0.91 |
| 2-chloro-4,6-bis(isopropyl-amino)-s-triazine | 0.25 |

### Example 3

A 22% by weight solution of cyanuric chloride in acetone was introduced to a continuous reactor consisting of two back mixed stages. Sufficient isopropylamine was added to the reactor to give a molar ratio of cyanuric chloride:isopropylamine of 1.05:1. Sufficient sodium hydroxide was added as a 20% weight aqueous solution to maintain the pH of the aqueous phase of the product emerging from the reactor in the range of 8 to 9. The reaction temperature was 10°C and the total average residence time was 2.5 hours.

The reactor product was then introduced to a hydrolysis consisting of a continuous, back mixed reactor. The average residence time was 1.5 hours at 35°C. Sufficient sodium hydroxide was added as a 20% by weight solution to maintain the pH in the range of 7 to 10. The aqueous phase was separated at 35°C in a continuous separator operated with a residence time of 0.5 hours. The organic phase from the separator was fed to a two stage continuous reactor operated at 35 to 45°C. The total average residence time in this reactor was 4.5 hours. A 5 to 10% molar excess of ethylamine based on 2,6 - dichloro - 4 - isopropylamino - s - triazine was added, as well as sufficient sodium hydroxide to maintain the pH of the aqueous phase of the reactor effluent in the range of 9 to 9.5. The crude product from this process was analysed by gas-liquid chromatography with the following result:

| Component | Percent by Weight |
|---|---|
| 2-chloro-4-ethylamino-6-isopropylamino-s-triazine | 98.2 |
| 2-chloro-4,6-bis(ethylamino)-s-triazine | 0.3 |
| 2-chloro-4,6-bis(isopropyl-amino)-s-triazine | 1.0 |

### Example 4

Using the apparatus and general procedure described in Example 1, a 30.5% by weight solution of cyanuric chloride in acetone was charged into a batch reaction vessel. A less than stoichiometric amount of isopropylamine (the molar ratio of cyanuric chloride to isopropylamine was 1.04:1) was added over a period of one hour and then sufficient 20% by weight sodium hydroxide in water was added over a period of 40 minutes to bring the pH of the aqueous phase to 8.0. The temperature of the reaction mixture during both reaction steps was controlled at 10°C. Hydrolysis of the excess cyanuric chloride was effected by maintaining a pH of 6.0 and heating the reaction mixture to 35°C for a period of 0.5 hours. Aqueous sodium hydroxide solution was added as necessary to maintain the pH of the reaction mixture at 6.0. At the conclusion of this reaction step, the pH was increased to 8.0 and ethylamine as a 68% solution in water was added over a period of 30 minutes. Sufficient 20% by weight sodium hydroxide solution was added over a period of 45 minutes to produce a pH of 8.5. The temperature of the reaction mixture was maintained at 35°C during this reaction sequence. Crude atrazine was isolated from the reaction mixture by precipitation with water. The molar yield of atrazine was 94.3% based on the isopropylamine starting material. The composition of the crude atrazine was determined by gas-liquid chromatography and is given below.

| Compound | Composition % by Weight |
|---|---|
| 2-chloro-4,6-bis(ethylamino)-s-triazine | 1.0 |
| 2-chloro-4,6-bis(isopropyl-amino)-s-triazine | 0.3 |
| 2-chloro-4-ethylamino-6-isopropylamino-s-triazine | 96.3 |

## Claims

1. A process for the preparation of 2 - chloro - 4 - ethylamino - 6 - isopropylamino - s - triazine in which cyanuric chloride is reacted in a first amination step with either isopropylamine or ethylamine to give either 2,6 - dichloro - 4 - isopropylamino - s - triazine or 2,6 - dichloro - 4 - ethylamino - s - triazine respectively, which is subsequently reacted in a second amination step with either ethylamine or isopropylamine respectively, both reactions being performed in a mixed acetone/water solvent and in the presence of an acid binding agent, characterised in that in the first amination step the molar ratio of cyanuric chloride to alkylamine is at least 1.04: 1, and that subsequent to the first amination step but prior to the second amination step, the cyanuric chloride remaining in the reaction mixture is hydrolysed, using a sufficient quantity of a base to maintain the pH of the reaction mixture in the range of 6 to 11, to a water-soluble salt of monohydroxydichlorotriazine.

2. A process for the preparation of 2 - chloro - 4 - ethylamino - 6 - isopropylamino - s - triazine in which cyanuric chloride is reacted in a first amination step with isopropylamine to give 2,6 - dichloro - 4 - isopropylamino - s - triazine which is subsequently reacted in a second amination step with ethylamine, both reactions being performed in a mixed acetone/water solvent and in the presence of an acid binding agent which is an alkali metal hydroxide, characterised in that in the first amination step the molar ratio of cyanuric chloride to isopropylamine is at least 1.04:1, and that subsequent to the first amination step but prior to the second amination step, the cyanuric chloride remaining in the reaction mixture is hydrolysed using a sufficient quantity of a base to maintain the pH of the reaction mixture in the range 6 to 11, to a water-soluble salt of monohydroxydichlorotriazine.

3. A process according to either claim 1 or claim 2, characterised in that in the first amination step in the molar ratio of cyanuric chloride to alkylamine is in the range 1.04:1 to 1.08:1.

4. A process according to any one of claims 1 to 3 characterised in that the base used to hydrolyse cyanuric chloride remaining in the reaction mixture after the first amination step is an alkali or alkaline earth metal hydroxide.

5. A process according to claim 4, characterised in that the base used to hydrolyse cyanuric chloride remaining in the reaction mixture after the first amination step is sodium hydroxide.

6. A process according to any one of claims 1 to 5, characterised in that the hydrolysis of cyanuric chloride is carried out at a pH in the range of 7 to 10.

7. A process according to any one of claims 1 to 6, characterised in that the hydrolysis of cyanuric chloride is carried out at a temperature in the range of 35 to 50°C.

8. A process according to any one of claims 1 to 7, characterised in that after the hydrolysis reaction and prior to the second amination step, at least some of the salt of monohydroxydichlorotriazine is removed from the reaction mixture by separating off the aqueous phase.

## Revendications

1. Un procédé pour la préparation de 2 - chloro - 4 - éthylamino - 6· - isopropylamino - s - triazine, selon lequel on fait réagir du chlorure cyanurique dans une première étape d'amination avec de l'isopropylamine ou de l'éthylamine pour obtenir de la 2,6 - dichloro - 4 - isopropylamino - s - triazine ou de la 2,6 - dichloro - 4 - éthylamino - s - triazine, respectivement, que l'on fait réagir ensuite dans une deuxième étape d'amination avec de l'éthylamine ou de l'isopropylamine, respectivement, les deux réactions étant conduites dans un solvant mixte acétone/eau et en présence d'un agent de fixation des acides, caractérisé en que dans la première étape d'amination le rapport molaire du chlorure cyanurique à l'alcoylamine est d'au moins 1,04:1 et qu'après la première étape d'amination, mais avant la deuxième étape d'amination, le chlorure cyanurique restant dans le mélange de réaction est hydrolysé, en utilisant uner quantité suffisante d'une base pour maintenir le pH du mélange réactionnel dans l'intervalle de 6 à 11, de façon à donner un sel soluble dans l'eau de monohydroxydichlorotriazine.

2. Un procédé pour la préparation de 2 - chloro - 4 - éthylamino - 6 - isopropylamino - s - triazine, selon lequel on fait réagir du chlorure cyanurique dans une première étape d'amination avec de l'isopropylamine pour obtenir de la 2,6 - dichloro - 4 - isopropylamino - s - triazine qui est ensuite mise à réagir dans une deuxième étape d'amination avec de l'éthylamine, les deux réactions étant conduites dans un solvant mixte acétone/eau et en présence d'un agent de fixation des acides qui est un hydroxyde de métal alcalin, caractérisé en ce que dans la première étape d'amination le rapport molaire du chlorure cyanurique à l'isopropylamine est d'au moins 1,04:1 et qu'après la première étape d'amination, mais avant la deuxième étape d'amination, on hydrolyse le chlorure cyanurique restant dans le mélange de réaction en utilisant une quantité suffisante d'une base pour maintenir le pH du mélange réactionnel dans l'intervalle de 6 à 11, de façon à obtenir un sel soluble dans l'eau de monohydroxydichlorotriazine.

3. Un procédé selon l'une des revendications 1 et 2, caractérisé en ce que dans la première étape d'amination le rapport molaire du chlorure cyanurique à l'alcoylamine est compris entre 1,04:1 et 1,08:1.

4. Un procédé selon l'une des revendications 1 à 3, caractérisé en ce que la base utilisée pour hydrolyser le chlorure cyanurique restant dans le mélange de réaction après la première étape d'amination est un hydroxyde de métal alcalin ou alcalino-terreux.

5. Un procédé selon la revendication 4, caracterisé en ce que la base utilisée pour hydrolyser le chlorure cyanurique restant dans le mélange de réaction après la première étape d'amination est de l'hydroxyde de sodium.

6. Un procédé selon l'une des revendications 1 à 5, caractérisé en ce que l'hydrolyse du chlorure cyanurique est effectuée à un pH compris entre 7 et 10.

7. Un procédé selon l'une des revendications 1 à 6, caractérisé en ce que l'hydrolyse du chlorure cyanurique est effectuée à une temperature comprise entre 35 et 50°C.

8. Un procédé selon l'une des revendications 1 à 7, caractérisé en ce qu'après la réaction d'hydrolyse et avant la deuxième étape d'amination, on élimine du mélange de réaction au moins une partie du sel de monohydroxydichlorotriazine en séparant la phase aqueuse.

**Patentansprüche**

1. Verfahren zur Herstellung von 2 - Chlor - 4 - äthylamino - 6 - isopropylamino - s - triazin, wobei Cyanursäurechlorid in einer ersten Aminierungsstufe entweder mit Isopropylamin oder mit Äthylamin zu 2,6 - Dichlor - 4 - isopropylamino - s - triazin bzw. 2,6 - Dichlor - 4 - äthyl amino - s - triazin umgesetzt wird, das anschliessend in einer zweiten Aminierungsstufe mit Äthylamin bzw. Isopropylamin umgesetzt wird, wobei beide Umsetzungen in einem Mischlösungsmittel aus Aceton und Wasser und in Anwesenheit eines Säurebindemittels durchgeführt werden, dadurch gekennzeichnet, dass in der ersten Aminierungsstufe das Molverhältnis von Cyanursäure-chlorid zu Alkylamin mindestens 1,04:1 beträgt, und dass anschliessend an die erste Aminierungsstufe, jedoch vor der zweiten Aminierungsstufe, das im Reaktionsgemisch verbleibende Cyanursäurechlorid unter Verwendung einer Menge einer Base, die dazu ausreicht, den pH-Wert des Reaktionsgemisches im Bereich von 6 bis 11 zu halten, zu einem wasserlöslichen Salz von Monohydroxydichlortriazin hydrolysiert wird.

2. Verfahren zur Herstellung von 2 - Chlor - 4 - äthylamino - 6 - isopropylamino - s - triazin, wobei Cyanursäurechlorid in einer ersten Aminierungsstufe mit Isopropylamin zu 2,6 - Dichlor - 4 - isopropylamino - s - triazin umgesetzt wird, das anschliessend in einer zweiten Aminierungsstufe mit Äthylamin umgesetzt wird, wobei beide Umsetzungen in einem Mischlösungsmittel aus Aceton und Wasser und in Anwesenheit eines Säurebindemittels, das ein Alkalimetallhydroxyd ist, durchgeführt werden, dadurch gekennzeichnet, dass in der ersten Aminierungsstufe das Molverhältnis von Cyanursäurechlorid zu Isopropylamin mindestens 1,04:1 beträgt, und das anschliessend an die erste Aminierungsstufe, jedoch vor der zweiten Aminierungsstufe das im Reaktionsgemisch verbleibende Cyanursäurechlorid unter Verwendung einer Menge einer Base, die dazu ausreicht, den pH-Wert des Reaktionsgemisches im Bereich von 6 bis 11 zu halten, zu einem wasserlöslichen Salz von Monohydroxydichlortriazin hydrolysiert wird.

3. Verfahren nach Anspruch 1 oder 2, dadurch gekennzeichnet, dass in der ersten Aminierungsstufe das Molverhältnis Cyanursäurechlorid zu Alkylamin im Bereich von 1,04:1 bis 1,08:1 liegt.

4. Verfahren nach einem der Ansprüche 1 bis 3, dadurch gekennzeichnet, dass die zum Hydrolysieren des nach der ersten Aminierungsstufe im Reaktionsgemisch verbleibenden Cyanursäurechlorids verwendete Base ein Alkali- oder Erdalkalimetallhydroxyd ist.

5. Verfahren nach Anspruch 4, dadurch gekennzeichnet, dass die zum Hydrolysieren des nach der ersten Aminierungsstufe im Reaktionsgemisch verbleibenden Cyanursäurechlorids verwendete Base Natriumhydroxid ist.

6. Verfahren nach einem der Ansprüche 1 bis 5, dadurch gekennzeichnet, dass die Hydrolyse von Cyanursäurechlorid bei einem pH-Wert im Bereich von 7 bis 10 durchgeführt wird.

7. Verfahren nach einem der Ansprüche 1 bis 6, dadurch gekennzeichnet, dass die Hydrolyse von Cyansäurechlorid bei einer Temperatur im Bereich von 35 bis 50°C durchgeführt wird.

8. Verfahren nach einem der Ansprüche 1 bis 7, dadurch gekennzeichnet, dass dem Reaktionsgemisch nach der Hydrolysereaktion und vor der zweiten Aminierungsstufe mindestens etwas Salz von Monohydroxydichlortriazin durch Abtrennen der wässrigen Phase entzogen wird.